# EUROPEAN PATENT APPLICATION

(11) **EP 2 756 852 A1**
(43) Date of publication of application: **23.07.2014**
(21) Application number: 13152187.4
(22) Date of filing: 22.01.2013
(51) Int. Cl.: A61K 47/48, A61P 33/06

(54) **Heparin-lipidic nanoparticle conjugates**

(71) Applicant: Centre de Recerca en Salut Internacional de Barcelona, 08036 Barcelona (ES); Fundació Institut de Bioenginyeria de Catalunya (IBEC), 08028 Barcelona (ES)
(72) Inventor: Fernández Busquets, Xavier, 08036 BARCELONA (ES); Azevedo Silva Marques, Maria Joana, 08036 BARCELONA (ES); Moles Meler, Ernesto, 08036 BARCELONA (ES)
(74) Representative: ZBM Patents - Zea, Barlocci & Markvardsen

(57) **Abstract**

These conjugates comprise 1,2-dioleoyl-3-trimethylammonium-propane (DOTAP) and heparin or a pharmaceutically acceptable salt thereof, wherein: the DOTAP is in a percentage by weight comprised from 1 to 30%; the heparin is conjugated to the liposome; and the molar ratio DOTAP:heparin is comprised from 15:1 to 1:20, and the weight ratio between the liposome total lipid content and heparin is comprised from 50:1 to 5:1.

## Description

The present invention refers to the field of medicine. In particular, the present invention provides heparin-lipidic nanoparticle conjugates which are useful in the treatment of malaria as well as in the drug delivery of other antimalarial drugs.

### BACKGROUND ART

Malaria is a mosquito-borne infectious disease of humans and other animals caused by protists (a type of microorganism) of the genus *Plasmodium.* It begins with a bite from an infected female mosquito (*Anopheles* mosquito), which introduces the protists via its saliva into the circulatory system, and ultimately to the liver where they mature and reproduce before being released into the blood where they parasitise erythrocytes and start the blood cycle. The disease causes symptoms that typically include fever and headache, which in severe cases can progress to coma or death. Malaria is widespread in tropical and subtropical regions in a broad band around the equator, including much of Sub-Saharan Africa, Asia, and the Americas.

Five species of *Plasmodium* can infect and be transmitted by humans. The vast majority of deaths are caused by *P. falciparum* while *P. vivax, P. ovale*, and *P. malariae* cause a generally milder form of malaria that is rarely fatal. The zoonotic species *P. knowlesi*, prevalent in Southeast Asia, causes malaria in macaques but can also cause severe infections in humans. Malaria is prevalent in tropical and subtropical regions because rainfall, warm temperatures, and stagnant waters provide habitats ideal for mosquito larvae. Disease transmission can be reduced by preventing mosquito bites by distribution of mosquito nets and insect repellents, or with mosquito-control measures such as spraying insecticides and draining standing water.

Drugs against malaria act at different stages of the parasite life cycle. Blood schizontocidal drugs (suppressive or clinical) attack parasites inside erythrocytes, preventing or ending clinical attack. Gametocytocidal drugs destroy sexual forms in human, decreasing transmission. Tissue schizonticidal drugs (causal, true) act on early stages of parasite development in liver, before the release of merozoites into blood.

Hypnozoitocidal drugs kill dormant hypnozoites in liver, preventing relapses of infection. Finally, sporontocidal drugs inhibit development of oocysts in mosquito, decreasing malaria transmission.

Among such therapeutic approaches, heparin has been extensively used as an adjunct treatment of severe malaria. Although this treatment has been successful in the majority of cases, the use of heparin was discontinued due to the occurrence of serious side effects such as intracranial bleeding.

Many efforts have been made to develop further drugs useful in the treatment of malaria. In this regard, the knowledge of the pathology of malaria and the underlying mechanisms leading to severe disease has greatly increased in the last 15 years and new findings have created new possibilities for treatment. The anti-malaria drugs available today are all directed toward the parasite itself. However, a great part of such drugs (primaquine, chloroquine, mefloquine, and quinine, among others) show side-effects such as dysphagia, nausea, dizziness, headache, blurred vision and pruritos, can cause hemolysis in G6PD-deficient patients, and cinchonism (which comprises tinnitus, deafness, headache, nausea and visual disturbance).

In spite of the efforts made until now, there is still the need of further therapeutical approaches which can efficiently treat malaria.

### SUMMARY OF THE INVENTION

The present inventors have developed a heparin-lipidic nanoparticle conjugate efficient in the treatment of malaria either alone or encapsulating therein a further antimalarial drug. In particular, the inventors have found that the design of the heparin-nanoparticle conjugate of the invention has to show a specific "equilibrium": there must be an amount of positive charge in the lipidic nanoparticle in such a way that the amount of conjugated heparin (which is negatively charged) is enough to provide the intended therapeutic or targetting effect, but without saturating the nanoparticle's membrane because, in this scenario, there would be a high risk that the heparin was delivered prior to contact with the infected cell, with the subsequent hemorrhagic problems that would occur. On the other hand, the amount of positive charge on the surface of the nanoparticle has not to hinder the approach of the conjugate to the *Plasmodium* infected cell surface (which is also positively-charged) as well as the subsequent heparin or antimalarial delivery by membrane fusion.

The inventors have found that the nanoparticle of the invention, including 1,2-dioleoyl-3-trimethylammonium-propane (hereinafter referred as DOTAP) as one of the components of the nanoparticle membrane, at a specific amount, and ratio vs. heparin, shows the specific "equilibrium" mentioned above because, as the experimental data show below, the heparin can effectively exhibit both the therapeutic and targetting ability.

It has been further found that DOTAP is not toxic for healthy cells up to a concentration of 200 µM, which is more higher than the amount included in the nanoparticles of the invention. This means that the lipidic conjugate of the invention is highly specific for *Plasmodium* infected cells, becoming a promising candidate for the treatment of malaria.

In addition, the inventors have found that with the specific ratio between the total lipid content of the nanoparticle and the heparin used, there is no risk of oversaturating the nanoparticle's surface, thus minimizing the possible "uncontrolled" delivered of the heparin prior to get to the infected cells.

Thus, in a first aspect the present invention provides a lipidic nanoparticle comprising 1,2-dioleoyl-3-trimethylammonium-propane (DOTAP), the nanoparticle being conjugated to heparin or a pharmaceutically or veterinary acceptable salt thereof, wherein
the DOTAP is in a percentage by weight comprised from 1 to 30% with respect to the lipidic nanoparticle,
the molar ratio DOTAP:heparin is comprised from 15:1 to 1:20, and the molar ratio between the total lipid content of the lipidic nanoparticle and heparin is comprised from 50:1 to 5:1.

As it has been stated above, heparin has been widely used as anti-malarial agent,but said treatment had to be discontinued due to the disruption of the coagulation cascade which led to haemorrhagic side-effects (due to the relatively high amounts of heparin required to obtain the therapeutic effect). Conjugating heparin to the nanoparticle of the invention, such glycosamylaminoglycan is "masked" in such a way that while the conjugated reaches the infected cell where it will exert its therapeutic activity, heparin is not in soluble form and thus its capacity to interact with antithrombin III is reduced, not being inhibited the coagulation cascade. In this way, administering the conjugate of the invention by any route, the heparin will not exert its action until reaching its target (the *Plasmodium* infected cell). Due to the "masking" effect provided by the lipidic nanoparticle and such site-directed specificity to the infected cells, the amount of free heparin with anticoagulant activity can be substantially reduced, being minimized the problems of haemorrhage and internal bleeding already disclosed in the prior art.

On the other hand, the lipidic nanoparticle of the invention first has to reach the infected cell and, then, the heparin has to be delivered to exert its therapeutic effect. In this regard, it has been found that the composition of the lipidic nanoparticle is such that it does neither significantly affect the heparin's targetting ability against *Plasmodium*-infected cells nor the delivery of heparin within the infected cell, in order to exert its therapeutic effect, as it is shown below.

Due to the special features of the nanoparticle conjugate of the invention, it can be used to formulate pharmaceutical or veterinary compositions.

Thus, in a second aspect the present invention provides a pharmaceutical or veterinary composition comprising a therapeutically effective amount of the lipidic nanoparticle as defined above, together with other appropriate pharmaceutically or veterinary acceptable excipients and/or carriers.

As it is shown below, the conjugate of the invention is effective in the treatment of malaria.

Thus, in a third aspect the present invention provides a lipidic nanoparticle according to the first aspect of the invention for use as a medicine. This aspect can also be formulated as a method for the treatment of a disease which comprises administering to mammals in need of such treatment a therapeutically effective amount of the heparin-lipidic nanoparticle conjugate of the present invention, together with one or more appropriate pharmaceutically acceptable excipients or carriers.

In a fourth aspect, the present invention provides a lipidic nanoparticle as defined above for use in the treatment of malaria. This aspect can be formulated as the use of a lipidic nanoparticle as defined above for the manufacture of a medicament for the treatment of malaria. This aspect can also be formulated as a method for the treatment of malaria comprising administering a therapeutically effective amount of the heparin-lipidic conjugate as defined above, together with pharmaceutically acceptable excipients or carriers, in a subject in need thereof, including a human.

On the other hand, the delivery of encapsulated drugs via membrane fusion is a highly desirable goal because of protection of the cargo from degradation, potential for targeting and decrease of drug toxicity.

As it is illustrated below, the inventors of the present invention have encapsulated within the lipidic nanoparticle conjugate of the first aspect an antimalarial drug, primaquine.

Primaquine has long been used with success against hepatic forms of malaria parasites, although toxic effects are frequently evident. Hence, there is an urgent need for a more effective treatment.

The experimental data show that the conjugate of the invention is a valuable drug delivery system, because primaquine exerts its antamalarial effect in infected *Plasmodium* cells. Particularly, it is observed that a lipidic nanoparticle including DOTAP and primaquine shows a poor antimalaric effect in comparison with the effect obtained when heparin is conjugated to the surface of the liposome. In this regard, there is a reduction in the number of infected cells of more than seven times when a heparin-lipidic nanoparticle conjugate of the invention encapsulating primaquine is used. This finding underscores the novel and significant medical advance achieved by the present invention.

This experimental data supports some aspects: (a) the lipidic nanoparticle surface with such amount of DOTAP and DOTAP:heparin ratio allows its approach to the surface of the infected cell; (b) due to the amount of heparin conjugated on the surface of the nanoparticle there is an intimate contact between the membrane of the lipidic nanoparticle and the membrane of the *Plasmodium* infected cell during a period of time enough to ensure the membrane fusion and, hence, the delivery of the antimalarial drug encapsulated therein; (c) the encapsulation of the antimalarial drug within the lipidic nanoparticle does not negatively affect the behaviour of the nanoparticle; and (d) the encapsulation of the antimalarial drug within the heparin-lipidic nanoparticle conjugate does not negatively affect the therapeutic effect of the drug.

The encapsulation of the drug in a lipidic nanoparticle of the present invention can enhance the therapeutic index of the bioactive agent, by buffering the agent's toxicity, and also can reduce the rate at which a bioactive agent is cleared from the circulation of animals. Accordingly, lipidic nanoparticle formulations of bioactive agents can mean that less of the agent needs to be administered to achieve the desired effect.

In view of the above, therefore, the present invention provides, in a further aspect, the use of the lipidic nanoparticle as defined above as drug delivery system.

### BRIEF DESCRIPTION OF THE DRAWINGS

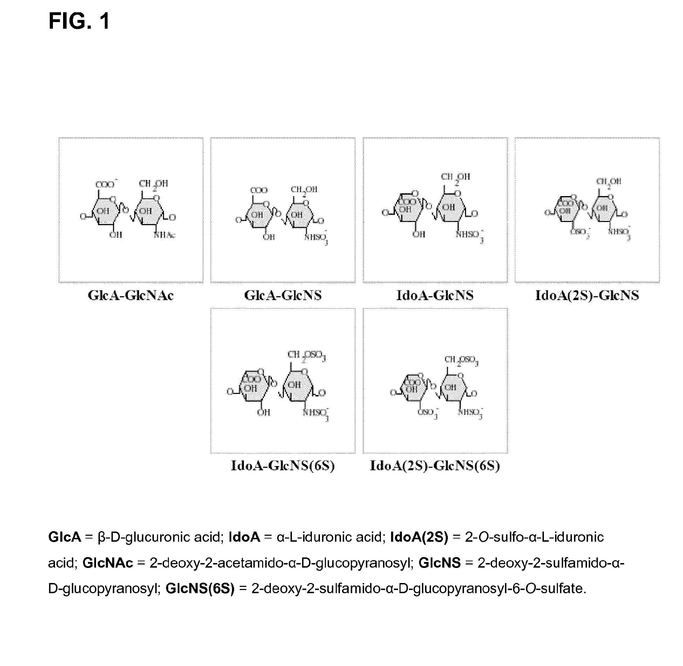

FIG. 1 is a representation of the main disaccharides that can occur in the heparin referred in the present invention.

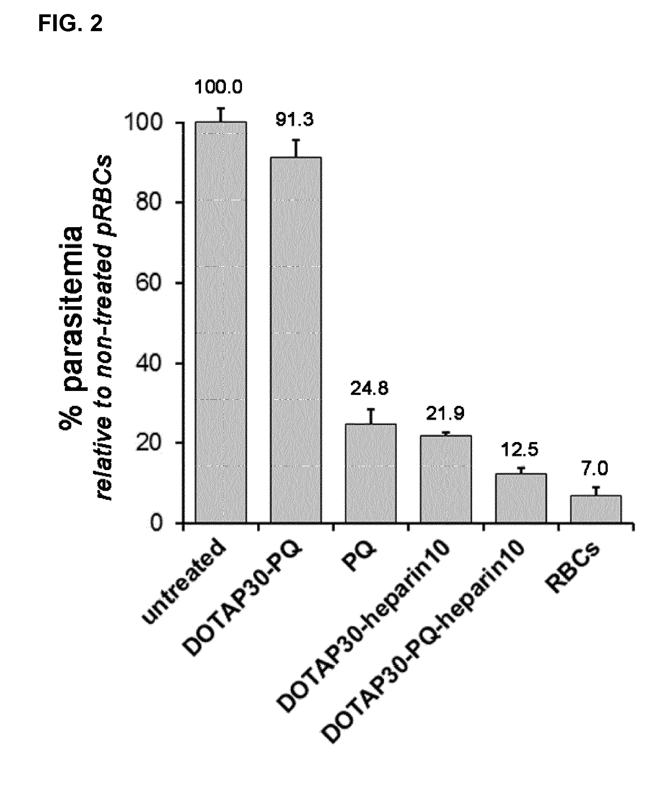

FIG. 2 corresponds to a bar representation showing the parasitemia effect of several lipidic suspensions. The parasitemia (vertical axis) is expressed in %, and is expressed with respect to non-treated *Plasmodium* infected red blood cells. In the horizontal axis, the different suspensions as specified: untreated = non-treated pRBC sample; DOTAP30-PQ = pRBC treated with a primaquine-containing liposome suspension, the liposome being formed by the molar ratio DOTAP:cholesteroI:DOPC 30:20:50, such that the final concentrations in the culture are 3 µM for primaquine and 15 µM for DOTAP; PQ = pRBC treated with free primaquine at a concentration in the culture of 3 µM; DOTAP30-heparin10 = pRBC treated with a heparin-conjugated liposome suspension without primaquine, the liposome being formed by the molar ratio DOTAP:cholesterol:DOPC 30:20:50, such that the final concentrations in the culture are 15 µM for DOTAP and 10 µM for heparin (i.e., molar ratio 1.5:1); DOTAP30-PQ-heparin10 = pRBC treated with a heparin-conjugated liposome suspension containing primaquine, the liposome being formed by the molar ratio DOTAP:cholesterol:DOPC 30:20:50, such that the final concentrations in the culture are 3 µM for primaquine, 15 µM for DOTAP, and 10 µM for heparin (i.e. DOTAP:heparin molar ratio 1.5:1); RBCs = control sample without pRBCs.

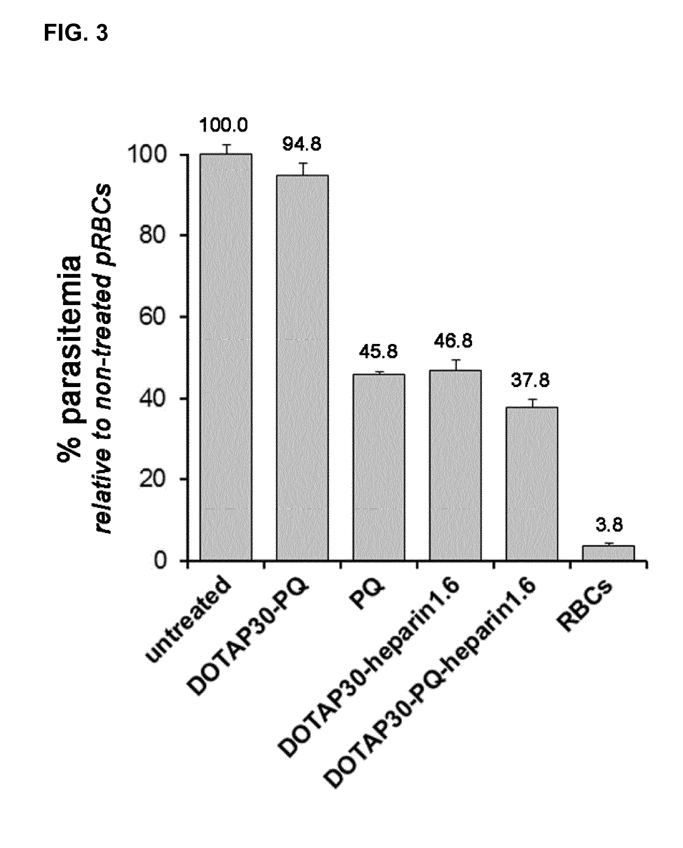

FIG. 3 corresponds to a bar representation showing the parasitemia effect of several lipidic suspensions. The parasitemia (vertical axis) is expressed in %, and is expressed with respect to non-treated *Plasmodium* infected red blood cells. In the horizontal axis, the different suspensions as specified: untreated = non-treated pRBC sample; DOTAP30-PQ = pRBC treated with a primaquine-containing liposome suspension, the liposome being formed by the molar ratio DOTAP:cholesterol:DOPC 30:20:50, such that the final concentrations in the culture are 3 µM for primaquine and 15 µM for DOTAP; PQ = pRBC treated with free primaquine at a concentration in the culture of 3 µM; DOTAP30-heparin1.6 = pRBC treated with a heparin-conjugated liposome suspension without primaquine, the liposome being formed by the molar ratio DOTAP:cholesterol:DOPC 30:20:50, such that the final concentrations in the culture are 15 µM for DOTAP and 1.6 µM for heparin (i.e. the DOTAP:heparin molar ratio is about 9.4:1); DOTAP30-PQ-heparin1.6 = pRBC treated with a heparin-conjugated liposome suspension containing primaquine, the liposome being formed by the molar ratio DOTAP:cholesterol:DOPC 30:20:50, such that the final concentrations in the culture are 3 µM for primaquine, 15 µM for DOTAP, and 1.6 µM for heparin (i.e. the DOTAP:heparin molar ratio is about 9.4:1); RBCs = control sample without pRBCs.

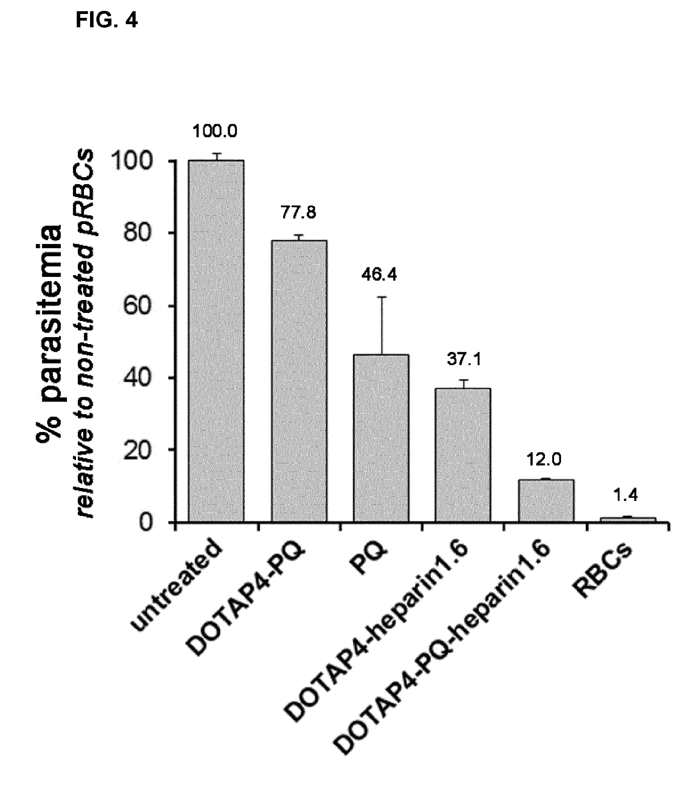

FIG. 4 corresponds to a bar representation showing the parasitemia effect of several lipidic suspensions. The parasitemia (vertical axis) is expressed in %, and is expressed with respect to non-treated *Plasmodium* infected red blood cells. In the horizontal axis, the different suspensions as specified: untreated = non-treated pRBC sample; DOTAP4-PQ = pRBC treated with a primaquine-containing liposome suspension, the liposome being formed by the molar ratio DOTAP:cholesterol:DOPC 4:20:76, such that the final concentrations in the culture are 3 µM for primaquine and 2 µM for DOTAP; PQ = pRBC treated with free primaquine at a concentration in the culture of 3 µM; DOTAP4-heparin1.6 = pRBC treated with a heparin-conjugated liposome suspension without primaquine, the liposome being formed by the molar ratio DOTAP:cholesterol:DOPC 4:20:76, such that the final concentrations in the culture are 2 µM for DOTAP and 1.6 µM for heparin (i.e. the DOTAP:heparin molar ratio is 1.25:1); DOTAP4-PQ-heparin1.6 = pRBC treated with a heparin-conjugated liposome suspension containing primaquine, the liposome being formed by the molar ratio DOTAP:cholesterol:DOPC 4:20:76, such that the final concentrations in the culture are 3 µM for primaquine, 2 µM for DOTAP, and 1.6 µM for heparin (i.e. the DOTAP:heparin molar ratio is about 1.25:1); RBCs = control sample without pRBCs.

### DETAILED DESCRIPTION OF THE INVENTION

In a first aspect, the present invention provides heparin-lipidic nanoparticle conjugates.

The term "percentage (%) by weight" refers to the percentage of each component of the nanoparticle conjugate in relation to the total weight.

The term "molar ratio" refers to the relation of moles of one compound in relation to the moles of another compound. In particular, in the present invention, the molar ratio DOTAP:heparin refers to the relation of moles of DOTAP vs the moles of heparin, whereas the molar ratio between the total lipid content of the lipidic nanoparticle and heparin refers to the relation of moles of total lipid content vs moles of heparin.

The term "nanoparticle" as used herein, refers to a particle with at least two dimensions at the nanoscale, particularly with all three dimensions at the nanoscale, where the nanoscale is the range about 1 nm to about 200 nm. Particularly, when the nanoparticle is substantially rod-shaped with a substantially circular cross-section, such as a nanowire or a nanotube, the "nanoparticle" refers to a particle with at least two dimensions at the nanoscale, these two dimensions being the cross-section of the nanoparticle.

The term "lipidic nanoparticle" as used herein, refers to a nanoparticle whose membrane is totally made of lipids. Suitable lipids include, without limitation, phospholipids such as phosphatidylcholines ("PC's"), phosphatidylethanolamines ("PE's"), phosphatidyilserines ("PS's"), phosphatidylglycerols ("PG's"), phosphatidylinositols ("PI's") and phosphatidic acids ("PA's"). Such phospholipids generally have two acyl chains, these being either both saturated, both unsaturated or one saturated and one unsaturated; said chains include, without limitation: myristate, palmitate, stearate, oleate, linoleate, linolenate, arachidate, arachidonate, behenate and lignocerate chains. Phospholipids can also be derivatized, by the attachment thereto of a suitable reactive group. Such a group is generally an amino group, and hence, derivatized phospholipids are typically phosphatidylethanolamines. The different moieties suited to attachment to PE's include, without limitation: acyl chains, useful for enhancing the fusability of liposomes to biological membranes; peptides, useful for destabilizing liposomes in the vicinity of target cells; biotin and maleimido moieties, useful for linking targeting moieties such as antibodies to liposomes; and various molecules such as gangliosides, polyalkylethers, polyethylene glycols and organic dicarboxylic acids. Other lipids which can constitute the membrane of the nanoparticle include, but are not limited to, cholesterol and DOPC.

In one embodiment, the lipidic nanoparticle is selected from the group consisting of liposomes and solid-lipid nanoparticle. Preferably, the lipidic nanoparticle is a liposome.

The term "solid lipid nanoparticle" refers to particles, typically spherical, with an average diameter between 10 to 1000 nanometers. Solid lipid nanoparticles possess a solid lipid core matrix that can solubilize lipophilic molecules. The lipid core is stabilized by surfactants (emulsifiers). The term lipid is used here in a broader sense and includes triglycerides (e.g. tristearin), diglycerides (e.g. glycerol bahenate), monoglycerides (e.g. glycerol monostearate), fatty acids (e.g. stearic acid), steroids (e.g. cholesterol), and waxes (e.g. cetyl palmitate). All classes of emulsifiers (with respect to charge and molecular weight) have been used to stabilize the lipid dispersion.

In the present invention, the term "liposome" is to be understood as a self-assembling structure comprising one or more lipid bilayers, each of which comprises two monolayers containing amphipathic lipid molecules oppositely oriented. Amphipathic lipids comprise a polar (hydrophilic) headgroup region covalently linked to one or two non-polar (hydrophobic) acyl chains. Energetically unfavorable contacts between the hydrophobic acyl chains and the surrounding aqueous medium induce the amphipathic lipid molecules to arrange themselves such that their polar headgroups are oriented towards the bilayer's surface, while the acyl chains reorient towards the interior of the bilayer. An energetically stable structure is thus formed in which the acyl chains are effectively shielded from coming into contact with the aqueous environment.

Liposomes can have a single lipid bilayer (unilamellar liposomes,"ULVs"), or multiple lipid bilayers (multilamellar liposomes,"MLVs"or"SPLVs"). Each bilayer surrounds, or encapsulates, an aqueous compartment. Given this encapsulation of aqueous volume within a protective barrier of lipid molecules, liposomes are able to sequester encapsulated molecules, e. g., nucleic acids, away from the degrading effects of factors, e. g., nuclease enzymes, present in the external environment.

Liposomes can have a variety of sizes, e. g., an average diameter as low as 25 nm or as high as 10,000 nm or more. Size is affected by a number of factors, e. g., lipid composition and method of preparation, well within the purview of ordinarily skilled artisans to determine and account for, and is determined by a number of techniques, such as quasi-elastic light scattering, also within the skilled person in the art knowledge.

Various methodologies, also well-known to those skilled in the art, such as sonication, or homogenization, and milling can be used to prepare liposomes of a smaller size from larger liposomes. Extrusion can be used to size reduce liposomes, that is to produce liposomes having a predetermined mean size by forcing the liposomes, under pressure, through filter pores of a defined, selected size. Tangential flow filtration can also be used to regularize the size of liposomes, that is, to produce a population of liposomes having less size heterogeneity, and a more homogeneous, defined size distribution.

Liposomes of this invention can be unilamellar, or oligolamellar, and can have a size equal to that of liposomes produced by any of the methods set forth hereinabove.

However, in embodiments of this invention, the liposomes are unilamellar liposomes having number average sizes between 100 and 200 nm.

In the present invention, the term "heparin" is to be understood as a highly sulfated glycosaminoglycan polymer with a molecular weight ranging from 3 kDa to 30 kDa, which belongs to the carbohydrates glycosaminoglycan family (which includes the closely related molecule heparan sulfate) and consists of a variably sulfated repeating disaccharide unit. The main disaccharide units that occur in heparin are shown in FIG. 1. The most common disaccharide unit is composed of a 2-O-sulfated iduronic acid and 6-O-sulfated, N-sulfated glucosamine, IdoA(2S)-GlcNS(6S). For example, this makes up 85% of heparins from beef lung and about 75% of those from porcine intestinal mucosa. In addition, there are the rare disaccharides containing a 3-O-sulfated glucosamine (GlcNS(3S,6S)) or a free amine group (GlcNH₃⁺). Under physiological conditions, the ester and amide sulfate groups are deprotonated and attract positively charged counterions to form a heparin salt. Preferably, the nanoparticle is conjugated to a heparin salt. More preferably, the heparin salt is sodium salt.

In one embodiment, the heparin is conjugated to the lipidic nanoparticle through electrostatic interactions between the positive charge of the lipidic nanoparticle membrane surface and the negative charge of the heparin.

In another embodiment, the heparin is conjugated to the lipidic nanoparticle via a covalent bound. In this embodiment, the nanoparticle further includes phosphatidylethanolamine (PE), and the covalent bound is formed via the NH₂ groups of PE and the carboxyl groups existing in the heparin molecule. The covalent bound is formed by dissolving the nanoparticles and heparin in a buffered solution of 2.5 mM N-(3-dimethylaminopropyl)-N'-ethylcarbodiimide hydrochloride (EDAC, Fluka) and 10 mM N-hydroxysuccinimide (NHS, Fluka). Finally, after an appropiate time of reaction at room temperature, nanoparticles can be purified by size, such as by size-exclusion chromatography.

The present inventors believe that with such covalent bond, heparin would be more strongly bound to the liposome, being reduced the amount of DOTAP, within the weight % range pointed out above.

In still another embodiment, part of the heparin amount is conjugated to the nanoparticle surface through electrostatic interactions, and the other part of the heparin amount is conjugated via a covalent bond.

In one embodiment, the heparin has a molecular weight comprised from 5-25 KDa or 10-20 KDa. Preferably, the heparin has a molecular weight comprised from 12 to 13 KDa.

In another embodiment, the weight percentage of DOTAP is comprised from 4 to 30%. In another embodiment, the weight percentage of DOTAP is comprised from 4 to 25%. In another embodiment, the weight percentage of DOTAP is comprised from 4 to 20%. In another embodiment, the weight percentage of DOTAP is comprised from 4 to 15%. In another embodiment, the DOTAP weight percentage is 1 %, 2%, 3%, 4%, 5%, 6%, 7%, 8%, 9%, 10%, 11 %, 12%, 13%, 14%, 15%, 16%, 17%, 18%, 19%, 20%, 21%, 22%, 23%, 24%, 25%, 26%, 27%, 28%, 29% or 30%.

In still another embodiment, the molar ratio DOTAP:heparin is comprised from 10:1 to 1:3. In another embodiment, the molar ratio DOTAP:heparin is comprised from 10:1 to 1:2. In another embodiment, the molar ratio DOTAP:heparin is comprised from 10:1 to 1:1. In another embodiment, the molar ratio DOTAP:heparin is comprised from 9:1 to 1:3. In another embodiment, the molar ratio DOTAP:heparin is comprised from 9:1 to 1:2. In another embodiment, the molar ratio DOTAP:heparin is comprised from 9:1 to 1:1. In another embodiment, the molar ratio DOTAP:heparin is comprised from 8:1 to 1:3. In another embodiment, the molar ratio DOTAP:heparin is comprised from 8:1 to 1:2. In another embodiment, the molar ratio DOTAP:heparin is comprised from 8:1 to 1:1. In another embodiment, the molar ratio DOTAP:heparin is comprised from 7:1 to 1:3. In another embodiment, the molar ratio DOTAP:heparin is comprised from 7:1 to 1:2. In another embodiment, the molar ratio DOTAP:heparin is comprised from 7:1 to 1:1. In another embodiment, the molar ratio DOTAP:heparin is comprised from 6:1 to 1:3. In another embodiment, the molar ratio DOTAP:heparin is comprised from 6:1 to 1:2. In another embodiment, the molar ratio DOTAP:heparin is comprised from 6:1 to 1:1. In another embodiment, the molar ratio DOTAP:heparin is comprised from 5:1 to 1:3. In another embodiment, the molar ratio DOTAP:heparin is comprised from 5:1 to 1:2. In another embodiment, the molar ratio DOTAP:heparin is comprised from 5:1 to 1:1. In another embodiment, the molar ratio DOTAP:heparin is comprised from 4:1 to 1:3. In another embodiment, the molar ratio DOTAP:heparin is comprised from 4:1 to 1:2. In another embodiment, the molar ratio DOTAP:heparin is comprised from 4:1 to 1:1. In another embodiment, the molar ratio DOTAP:heparin is comprised from 3:1 to 1:3. In another embodiment, the molar ratio DOTAP:heparin is comprised from 3:1 to 1:2. In another embodiment, the molar ratio DOTAP:heparin is comprised from 3:1 to 1:1. In another embodiment, the molar ratio DOTAP:heparin is comprised from 2:1 to 1:3. In another embodiment, the molar ratio DOTAP:heparin is comprised from 2:1 to 2:2. In another embodiment, the molar ratio DOTAP:heparin is comprised from 2:1 to 1:1. In another embodiment, the molar ratio DOTAP:heparin is comprised from 1:1 to 1:3. In another embodiment, the molar ratio DOTAP:heparin is comprised from 1:1 to 1:2. In another embodiment, the molar ratio DOTAP:heparin is 1:1. In another embodiment, the molar ratio DOTAP:heparin is selected from: 15:1, 2:1, 1.5:1, and 1:15, or, alternatively, it is comprised from 2:1 to 1:1 or it is comprised from 10:1 to 9:1.

In still another embodiment, the molar ratio between the total amount of lipid forming the membrane lipidic nanoparticle vs heparin is comprised from 50:1 to 10:1. In another embodiment, the molar ratio between the total amount of lipid forming the membrane lipidic nanoparticle vs heparin is comprised from 40:1 to 10:1. In still another embodiment, the molar ratio between the total amount of lipid forming the membrane lipidic nanoparticle vs heparin is comprised from 35:1 to 20:1. In still another embodiment, the molar ratio between the total amount of lipid forming the membrane lipidic nanoparticle vs heparin is comprised from 35:1 to 30:1. In another embodiment, the molar ratio between the total amount of lipid forming the membrane lipidic nanoparticle vs heparin is selected from the group consisting of: 35:1, 34:1, 33:1, 32:1, 31:1, 30:1, 29:1, 28:1, 27:1, 26:1, 25:1, 24:1, 23:1, 22:1, 21:1, and 20:1.

In one embodiment, the lipidic nanoparticle comprises cholesterol. In another embodiment, the lipidic nanoparticle comprises cholesterol in a percentage by weight comprised from 10 to 30. In another embodiment, the lipidic nanoparticle comprises a 20% by weight of cholesterol.

In another embodiment, the lipidic nanoparticle comprises 1,2-dioleoyl-sn-glycero-3-phosphatidylcholine (hereinafter also referred as "DOPC"). In one embodiment, DOPC is in a percentage by weight comprised from 40 to 89%.

In another embodiment, the lipidic nanoparticle comprises DOTAP at a percentage by weight from 4 to 30%, cholesterol in a 20 % by weight, and DOPC in a percentage by weight comprised from 50 to 76%.

In another embodiment, the lipidic nanoparticle is a liposome comprising 76% by weight of DOPC, 20% by weight of cholesterol, and 4% by weight of DOTAP.

In another embodiment, the lipidic nanoparticle is a liposome comprising 50% by weight of DOPC, 20% by weight of cholesterol, and 30% by weight of DOTAP.

In another embodiment, the lipidic nanoparticle is a liposome comprising 76% by weight of DOPC, 20% by weight of cholesterol, and 4% by weight of DOTAP, and the molar ratio DOTAP: heparin is 2:1, 1:5, it is comprised from 2:1 to 1:1.

In another embodiment, the lipidic nanoparticle is a liposome comprising 50% by weight of DOPC, 20% by weight of cholesterol, and 30% by weight of DOTAP, and the molar ratio DOTAP:heparin is 15:1, 1.5:1, or it is comprised from 10:1 to 9:1.

In another embodiment, the lipidic nanoparticle is a liposome comprising 76% by weight of DOPC, 20% by weight of cholesterol, and 4% by weight of DOTAP, the molar ratio DOTAP: heparin is 2:1, 1:5, or it is comprised from 2:1 to 1:1, and the heparin is conjugated to the surface liposome through electrostatic interactions.

In another embodiment, the lipidic nanoparticle is a liposome comprising 50% by weight of DOPC, 20% by weight of cholesterol, and 30% by weight of DOTAP, the molar ratio DOTAP: heparin is 15:1, 1.5:1, or it is comprised from 10:1 to 9:1, and the heparin is conjugated to the surface liposome through electrostatic interactions.

In another embodiment, the lipidic nanoparticle is a liposome comprising 76% by weight of DOPC, 20% by weight of cholesterol, and 4% by weight of DOTAP, the molar ratio DOTAP: heparin is 2:1, 1:5, or it is comprised from 2:1 to 1:1, the heparin is conjugated to the surface liposome through electrostatic, interactions, and the molecular weight of heparin is from 12 to 13 KDa.

In another embodiment, the lipidic nanoparticle is a liposome comprising 50% by weight of DOPC, 20% by weight of cholesterol, and 30% by weight of DOTAP, the molar ratio DOTAP: heparin is 15:1, 1.5:1, or it is comprised from 10:1 to 9:1, the heparin is conjugated to the surface liposome through electrostatic interactions, and the molecular weight of the heparin is from 12 to 13 KDa.

The nanoparticles of the invention are obtained following procedures well-known to the skilled person in the art, namely, the lipid film hydration method. In one embodiment, the nanoparticles are liposomes and are prepared by a process comprising: (a) preparing the liposomes following the lipid film hydration method; (b) adding a solution of heparin or a salt thereof; and, optionally, (c) purifying the resulting lipidic conjugate by size, such as by size-exclusion chromatography or ultracentrifugation.

In another embodiment, the lipidic nanoparticle of the invention encapsulates a drug, preferably an anti-malarial drug.

The term "anti-malarial drug" is to be understood as any drug designed to prevent or cure malaria. Such drugs may be used for some or all of the following: (a) treatment of malaria in individuals with suspected or confirmed infection; (b) prevention of infection in individuals visiting a malaria-endemic region who have no immunity (malaria prophylaxis); and (c) routine intermittent treatment of certain groups in endemic regions (intermittent preventive therapy). Illustrative non-limitative examples of antimalarial drugs are quinine (and related agents), chloroquine, amodiaquine, pyrimethamine, proguanil, sulfonamides, mefloquine, atovaquone, primaquine, artemisinin and derivatives, halofantrine, doxycycline, clindamycin, and heparin. Preferably, the anti-malarial drug is primaquine.

There are well-known methods in the state of the art to encapsulate a drug within the nanoparticle of the first aspect of the invention. Encapsulation of hydrophobic or water-soluble drugs into, respectively, the bilayer or the hydrophilic core, can be done with a variety of loading strategies (Maurer N. et al., "Developments in liposomal drug delivery Systems", Expert Opin Biol Ther, 2001, vol. 1(6), page 923-47), which include the pH gradient (Waterhouse D. N. et al., "Preparation, characterization, and biological analysis of liposomal formulations of vincristine", Methods Enzymol., 2005; vol. 391, page 40-57) and ammonium sulfate methods, and the direct drug entrapping simultaneously with liposome formation (Urbán P. et al., "Study of the efficacy of antimalarial drugs delivered inside targeted immunoliposomal nanovectors", Nanoscale Research Letters, 2011, vol. 6, page 620).

In another embodiment, the lipidic nanoparticle is a liposome comprising 76% by weight of DOPC, 20% by weight of cholesterol, and 4% by weight of DOTAP, and the liposome encapsulates primaquine.

In another embodiment, the lipidic nanoparticle is a liposome comprising 50% by weight of DOPC, 20% by weight of cholesterol, and 30% by weight of DOTAP, and the liposome encapsulates primaquine.

In another embodiment, the lipidic nanoparticle is a liposome comprising 76% by weight of DOPC, 20% by weight of cholesterol, and 4% by weight of DOTAP, and the molar ratio DOTAP: heparin is 2:1, 1:5, or it is comprised from 2:1 to 1:1, and the liposome encapsulates primaquine.

In another embodiment, the lipidic nanoparticle is a liposome comprising 50% by weight of DOPC, 20% by weight of cholesterol, and 30% by weight of DOTAP, and the molar ratio DOTAP:heparin is 15:1 or 1.5:1, and the liposome encapsulates primaquine.

In another embodiment, the lipidic nanoparticle is a liposome comprising 76% by weight of DOPC, 20% by weight of cholesterol, and 4% by weight of DOTAP, the molar ratio DOTAP: heparin is 2:1, 1:5, or it is comprised from 2:1 to 1:1, the heparin is conjugated to the surface liposome through electrostatic interactions, and the liposome encapsulates primaquine.

In another embodiment, the lipidic nanoparticle is a liposome comprising 50% by weight of DOPC, 20% by weight of cholesterol, and 30% by weight of DOTAP, the molar ratio DOTAP: heparin is 15:1 or 1.5:1, the heparin is conjugated to the surface liposome through electrostatic interactions, and the liposome encapsulates primaquine.

In another embodiment, the lipidic nanoparticle is a liposome comprising 76% by weight of DOPC, 20% by weight of cholesterol, and 4% by weight of DOTAP, the molar ratio DOTAP: heparin is 2:1, 1:5, or it is comprised from 2:1 to 1:1, the heparin is conjugated to the surface liposome through electrostatic interactions, its molecular weight is from 12 to 13 KDa, and the liposome encapsulates primaquine.

In another embodiment, the lipidic nanoparticle is a liposome comprising 50% by weight of DOPC, 20% by weight of cholesterol, and 30% by weight of DOTAP, the molar ratio DOTAP: heparin is 15:1 or 1.5:1, the heparin is conjugated to the surface liposome through electrostatic interactions, its molecular weight is from 12 to 13 KDa, and the liposome encapsulates primaquine.

In the present invention, the expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

In the present invention, the term "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio. Likewise, the term "veterinary acceptable" means suitable for use in contact with a non-human animal.

As it has been stated above, and supported by the experimental data provided below, the lipidic nanoparticle of the invention is effective in the treatment of malaria. As it has been stated above, malaria can be casued by five different species: *P. falciparum, P. vivax, P. ovale, P. malariae,* and *P. knowlesi.* Thus, the lipidic nanoparticle of the invention can be effective in the treatment or drug-targetting against any of such five species. Preferably, the *Plasmodium* species is *P. falciparum.*

The term "vector control" has to be understood as a mean that preventively decreases malaria and morbidity and mortality, by reducing the levels of transmission through mosquito. Here, the lipidic nanoparticle of the invention could be dissolved in a medium mimetizing the human blood, being the mosquitos would be attracted. Such solution including the nanoparticles of the invention could be dispensed in containers to be hung

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

### Material and Methods:

### Liposome preparation

Liposomes were prepared by the lipid film hydration method (Urbán P. et al., A nanovector with complete discrimination for targeted delivery to Plasmodium falciparum-infected versus non-infected red blood cells in Vitro J Control Release, 2011, vol. 151 (2), pages 202-211). Briefly, different lipid combinations were tested in order to establish a liposomal formulation with low hemolytic activity and low general cytotoxicity. Lipids (phosphatidylethanolamine, PE; 1,2-dioleoyl-3-trimethylammonium-propane, DOTAP; cholesterol; 1,2-dioleoyl-sn-glycero-3-phosphatidylcholine, DOPC, Avanti Polar Lipids Inc., Alabaster, AL, USA; all ≥ 99% purity according to thin layer chromatography analysis) were dissolved in chloroform:methanol (2:1 v/v) in a round-bottomed flask. Lipids were stored at -20°C at the following concentrations: PE 10 mg/ml, DOTAP 30 mM, cholesterol 30 mM, and DOPC 60.6 mM. Cholesterol was present as 20% molar ratio of total lipid, DOTAP and PE were used at the concentrations described and DOPC was making up the rest up to 100% (e.g. DOPC:DOTAP:cholesterol 50:30:20, DOPC:DOTAP:cholesterol 76:4:20, etc.). Organic solvents were removed by rotary evaporation under reduced pressure at 37 °C to yield a thin lipid film on the walls of the flask, and remaining solvent traces were eliminated by drying under N₂ flow for 30 min. The dry lipids were hydrated in 1 ml PBS at 37 °C to obtain a concentration of 10 mM total lipid and multilamellar liposomes were formed by 3 cycles of constant vortexing followed by bath sonication for 4 min each. Multilamellar liposomes were downsized to form uni- or oligolamellar vesicles by extrusion through 200-nm polycarbonate membranes (Poretics, Livermore, CA, USA) in an extruder device (LiposoFast, Avestin, Ottawa, Canada). Liposome size was determined by dynamic light scattering using a Zetasizer NanoZS90 (Malvern Ltd, Malvern, UK). Liposomes encapsulating the antimalarial drug primaquine were prepared by dissolving it in the hydration buffer at a concentration of 6 mM, 2,000 times larger than its final concentration in the *Plasmodium* culture, which was 3 µM. . Finally, liposomes were purified by molecular exclusion chromatography (Zeba desalt spin columns, Pierce Biotechnology). The filtration step removed the drug not encapsulated in liposomes; being the liposomal volume ≤10% of the total, the estimated drug concentration in the liposome suspension is 600 µM after this filtration step. Because the liposomes undergo a 1:200 dilution when added to the *Plasmodium* culture, the final primaquine concentration in the culture was 3 µM.

Once the liposomes were formed (either alone or encapsulating therein primaquine), heparin sodium salt from porcine intestinal mucosa (Sigma-Aldrich, 13,000 kDa mean molecular mass) dissolved in PBS was added at the required concentration and incubated for 1.5 h at room temperature with gentle stirring to obtain the desired DOTAP:heparin ratios. As an example: in a 30% DOTAP liposome suspension containing 10 mM lipid there was 3 mM DOTAP; for a molar ratio DOTAP:heparin 3:2 the solution will contain 2 mM heparin; since addition to *Plasmodium* cultures represents a 1:200 dilution, the final molar concentrations in the culture will be 15 µM for DOTAP and 10 µM for heparin.

In this way, the following lipidic nanoparticle suspensions were made:
- Lipidic nanoparticle suspension 1 = 76% DOPC, 20% cholesterol, and 4% DOTAP;
- Lipidic nanoparticle suspension 2= 50% DOPC, 20% cholesterol, and 30% DOTAP;
- Lipidic nanoparticle suspension 3= Lipidic nanoparticle 1 (76% DOPC, 20% cholesterol, and 4% DOTAP) including therein primaquine at a concentration of 600 µM (for a final concentration in the culture of 3 µM); ;
- Lipidic nanoparticle suspension 4= Lipidic nanoparticle 2 (50% DOPC, 20% cholesterol, and 30% DOTAP) including therein primaquine at a concentration of 600 µM (for a final concentration in the culture of 3 µM) and 15 µM for DOTAP;
- Lipidic nanoparticle suspension 5= Lipidic nanoparticle 2 (i.e., formed by the molar ratio DOTAP:cholesterol:DOPC 30:20:50) such that the final concentrations in the culture were 15 µM for DOTAP and 10 µM for heparin (i.e., molar ratio 1.5:1);
- Lipidic nanoparticle suspension 6= Lipidic nanoparticle 2 (i.e., formed by the molar ratio DOTAP:cholesterol:DOPC 30:20:50), such that the final concentrations in the culture were 3 µM for primaquine, 15 µM for DOTAP, and 10 µM for heparin (i.e. DOTAP:heparin molar ratio 1.5:1);
- Lipidic nanoparticle suspension 7= Lipidic nanoparticle 2 (i.e., formed by the molar ratio DOTAP:cholesterol:DOPC 30:20:50), such that the final concentrations in the culture were 15 µM for DOTAP and 1.6 µM for heparin (i.e. the DOTAP:heparin molar ratio is about 9.4:1);
- Lipidic nanoparticle suspension 8= Lipidic nanoparticle 2 (i.e., formed by the molar ratio DOTAP:cholesterol:DOPC 30:20:50), such that the final concentrations in the culture were 3 µM for primaquine, 15 µM for DOTAP, and 1.6 µM for heparin (i.e. the DOTAP:heparin molar ratio is about 9.4:1);
- Lipidic nanoparticle suspension 9= Lipidic nanoparticle 1 (formed by the molar ratio DOTAP:cholesterol:DOPC 4:20:76) including therein primaquine, such that the final concentrations in the culture were 3 µM for primaquine and 2 µM for DOTAP;
- Lipidic nanoparticle suspension 10= Lipidic nanoparticle 1 (formed by the molar ratio DOTAP:cholesterol:DOPC 4:20:76) such that the final concentrations in the culture were 2 µM for DOTAP and 1.6 µM for heparin (i.e. the DOTAP:heparin molar ratio is 1.25:1); and
- Lipidic nanoparticle suspension 11= Lipidic nanoparticle 1 (formed by the molar ratio DOTAP:cholesterol:DOPC 4:20:76), such that the final concentrations in the culture were 3 µM for primaquine, 2 µM for DOTAP, and 1.6 µM for heparin (i.e. the DOTAP:heparin molar ratio is about 1.25:1).

### Treatment of blood

Human blood group B, collected in citrate-phosphate-dextrose (CPD) buffer, was provided by the *Banc de Sang i Teixits* at the *Hospital Vall d'Hebron* in Barcelona (www.bancsang.net/ca). 10 mL of full blood was collected in 50-mL centrifuge tubes, spun for 5 min at 1,200 *g*, and plasma and buffy coat were removed. The resulting RBC pellet was resuspended in 40 mL of washing medium containing 10.4 g/l RPMI-1640 (Invitrogen), 25 mM HEPES, 100 µM hypoxanthine, 12.5 µg/mL gentamicin, 23.8 mM sodium bicarbonate, pH 7.2. After centrifugation at 1,200 *g* for 10 min the supernatant was discarded, and after another washing step in 15-mL tubes, the RBC pellet was taken up in one volume of Roswell Park Memorial Institute (RPMI) complete medium (washing medium supplemented with 0.5% Albumax, 0.2% glucose, 25 µg/mL gentamicin, 50 µg/mL hypoxanthine, and 2 mM glutamine), stored at 4 °C and used within 10 days (washed RBCs).

### Plasmodium falciparum cell culture and growth inhibition assays

The *P. falciparum* 3D7 strain (available from Bei Resources (http://www.beiresources.org/) 10801 University Boulevard, Manassas, Virginia, 20110-2209) was grown *in vitro* in group B human RBCs using previously described conditions (Cranmer,S.L. et al., 1997). Briefly, parasites (thawed from glycerol stocks) were cultured at 37 °C in Petri dishes containing RBCs in RPMI complete medium under a gas mixture of 92% N₂, 5% CO₂, and 3% O₂. Synchronized cultures were obtained by 5% sorbitol lysis (Lambros,C. et al., "Synchronization of Plasmodium falciparum erythrocytic stages in culture", J Parasitol., 1979,vol. 65, 418-420), and the medium was changed every 2 days maintaining 3% hematocrit. For culture maintenance, parasitemias were kept below 5% late forms by dilution with washed RBCs.

For growth inhibition assays, parasitemia was adjusted to 1.5% with more than 90% of parasites at ring stage after sorbitol synchronization. 150 µL of this *Plasmodium* culture was plated in 96-well plates and incubated in the presence of liposomes for 48 h in the conditions described above.

Parasitemia was determined by microscopic counting of blood smears, or by fluorescence-assisted cell sorting (FACS). Smears were fixed in methanol for a few seconds and then stained for ten minutes with Giemsa (Merck Chemicals, Germany) diluted 1:10 in Sorenson's Buffer, pH 7.2. For FACS analysis, each sample was diluted 1:125 in PBS and Syto 11 (0.5 mM in DMSO) was added to a final concentration of 0.5 µM. Samples were analyzed using an Epics XL flow cytometer (Coulter Corporation, Miami, Florida), set up with the standard configuration. Excitation of the sample was done using a standard 488 nm air-cooled argon-ion laser at 15 mW power. Forward scatter and side scatter were used to gate the RBC population. Syto 11 green fluorescence was collected in a logarithmic scale with a 550 dichroic long filter and a 525 band pass filter. The single-cell population was selected on a forward-side scatter scattergram, and the fluorescence from this population was analyzed. Parasitemia was expressed as the number of parasitized cells per 100 erythrocytes.

### Fluorescence microscopy

Heparin-liposomes conjugates obtained as described above were incubated with washed living *P. falciparum* cultures for 90 min at 37 °C with gentle stirring. After incubation cells were spun down 2× 2 min at 1,000 *g* and washed with PBS before processing for fluorescence microscopy. After washing, blood smears were prepared and cells were fixed in 1% paraformaldehyde. Parasite nuclei were stained with 4'6-diamino-2-phenylindole (DAPI, Invitrogen) and the RBC membrane was labeled with wheat germ agglutinin (WGA)-tetramethylrhodamine conjugate (Molecular Probes, Eugene, OR, USA). Slides were finally mounted with Prolong® Gold antifade reagent (Invitrogen), and analyzed with a Leica TCS SP5 laser scanning confocal microscope. DAPI, FITC, reflection (hemozoin), and WGA-rhodamine images were acquired sequentially using 405, 488, 488 and 561 laser lines, and emission detection ranges 415-480 nm, 500-550 nm, 480-500 nm, and 571-625 nm, respectively, with the confocal pinhole set at 1 Airy units. Bright field transmitted light images were acquired simultaneously. Images were acquired at 400 Hz in a 512 × 512 pixels format, 8× zoom, and pixel size of 60 × 60 nm.

### Unspecific cytotoxicity assays

Cells were seeded in 96-well plates at a density of 5000 cells/100 µl/well, and incubated for 24 hours at 37°C in Medium 199 with Earle's Salts, supplemented with L-glutamine and FBS (PAA Laboratories GmbH). After that time the medium was carefully removed with a pipette and 90 µL of new medium (without FBS in this case) were added, followed by 10 µL of the sample to be analysed, which consisted of a solution of DOTAP 200 µM in PBS. It is advisable to prepare the sample dilutions in a 9:1 proportion (9 µl media:1 µl sample). After incubating for either 24 or 48 h at 37°C, 10 µL of WST-1 reagent were added, and after incubating for 1 h at 37°C absorbance at 440 nm was measured.

Cytotoxicity was not observed for a DOTAP concentration up to 200 µM.

### Example 1: Antimalarial activity of lipidic nanoparticles 4-6

Parasitemia of *P.falciparum* 3D7 cultures was adjusted to 1.5% with more than 90% of parasites at ring stage after sorbitol synchronization. 150 µl of this *Plasmodium* culture was plated in 96-well plates and incubated in the presence of liposomes (50 µM total lipid as final concentration in the culture dish) for 48 h at 37°C under a gas mixture of 92% N₂, 5% CO₂, and 3% O₂. Parasitemia was determined by microscopic counting of blood smears, or by fluorescence-assisted cell sorting (FACS). The results are summarized in FIG. 2.

Whereas free 3 µM PQ had an antimalarial activity within its expected range (24.8% parasitemia), when the PQ was encapsulated in a liposome having as composition DOTAP:cholesterol:DOPC 30:20:50, the parasitemia substantially increases (to 91.3%), which would be indicative that the encapsulation of primaquine substantially reduces its antimalarial effect.

However, when PQ was encapsulated in a liposome with the same composition but conjugated to heparin the parasitemia was substantially reduced (about 7 times). This is indicative of the fact that the heparin-lipidic nanoparticle of the invention allows the encapsulation of antimalarial drug increasing its therapeutic activity.

On the other hand, when a heparin-lipidic nanoparticle consisting of a liposome with a composition DOTAP:cholesterol:DOPC 30:20:50 was added to the pRBC culture (DOTAP: 30%, molar ratio DOTAP:heparin 1.5:1), it was found that the parasitemia was reduced about four times when compared with the parasitemia effect after administering the lipidic nanoparticle comprising primaquine. This is indicative of the fact that the lipidic nanoparticle of the invention do not negatively affect the antimalarial activity of heparin.

### Example 2: antimalarial activity of lipidic nanoparticles 4, 7, and 8

Parasitemia of *P.falciparum* 3D7 cultures was adjusted to 1.5% with more than 90% of parasites at ring stage after sorbitol synchronization. 150 µl of this *Plasmodium* culture was plated in 96-well plates and incubated in the presence of liposomes (50 µM total lipid as final concentration in the culture dish) for 48 h at 37°C under a gas mixture of 92% N₂, 5% CO₂, and 3% O₂. Parasitemia was determined by microscopic counting of blood smears, or by fluorescence-assisted cell sorting (FACS). The results are summarized in FIG. 3.

Whereas free 3 µM PQ had an antimalarial activity within its expected range (45.8% parasitemia), when the PQ was encapsulated in a liposome having as composition DOTAP:cholesterol:DOPC 30:20:50, the parasitemia substantially increases (up to 94.8%), which would be indicative that the encapsulation of primaquine substantially reduces its antimalarial effect.

However, when PQ was encapsulated in a liposome with the same composition but conjugated to heparin, the parasitemia was substantially reduced. This is indicative of the fact that the heparin-lipidic nanoparticle of the invention allows the encapsulation of antimalarial drug increasing its therapeutic activity.

On the other hand, when a heparin-lipidic nanoparticle consisting of a liposome with a composition DOTAP:cholesterol:DOPC 30:20:50 was added to the pRBC culture (with DOTAP:heparin molar ratio: 9.4:1), it was found that the parasitemia was reduced to half when compared with the parasitemia effect after administering the lipidic nanoparticle comprising primaquine. This is indicative of the fact that the lipidic nanoparticle of the invention do not negatively affect the antimalarial activity of heparin.

### Example 3: antimalarial activity of lipidic nanoparticles 9-11

Parasitemia of *P.falciparum* 3D7 cultures was adjusted to 1.5% with more than 90% of parasites at ring stage after sorbitol synchronization. 150 µl of this *Plasmodium* culture was plated in 96-well plates and incubated in the presence of liposomes (50 µM total lipid as final concentration in the culture dish) for 48 h at 37°C under a gas mixture of 92% N₂, 5% CO₂, and 3% O₂. Parasitemia was determined by microscopic counting of blood smears, or by fluorescence-assisted cell sorting (FACS). The results are summarized in FIG. 4.

Whereas free 3 µM PQ had an antimalarial activity within its expected range (46.4% parasitemia), when the PQ was encapsulated in a liposome having as composition DOTAP:cholesterol:DOPC 4:20:76, the parasitemia substantially increases (up to 77.8%), which would be indicative that the encapsulation of primaquine substantially reduces its antimalarial effect.

However, when PQ was encapsulated in a liposome with the same composition but conjugated to heparin added at 1.6 µM as final concentration in the culture dish (before addition to the culture dish the liposome suspension contained 0.32 mM heparin and 0.4 mM DOTAP), the parasitemia was substantially reduced (to 12.0%). This is indicative of the fact that the heparin-lipidic nanoparticle of the invention allows the encapsulation of antimalarial drug increasing its therapeutic activity.

On the other hand, when a heparin-lipidic nanoparticle consisting of a liposome with a composition DOTAP:cholesterol:DOPC 4:20:76 was added to the pRBC culture (with DOTAP:heparin molar ratio: 1.25:1), it was found that the parasitemia was reduced to half when compared with the parasitemia effect after administering the lipidic nanoparticle comprising primaquine. This is indicative of the fact that the lipidic nanoparticle of the invention do not negatively effect the antimalarial activity of heparin.

### REFERENCES CITED IN THE APPLICATION

Cranmer,S.L. et al., " An alternative to serum for cultivation of Plasmodium falciparum in vitro", Trans. R. Soc. Trop. Med. Hyg., 1997, vol. 91, pages 363-365;
Lambros,C. et al., "Synchronization of Plasmodium falciparum erythrocytic stages in culture", J Parasitol., 1979,vol. 65, 418-420;
Maurer N. et al., "Developments in liposomal drug delivery Systems", Expert Opin Biol Ther, 2001, vol. 1(6), page 923-47;
Waterhouse D. N. et al., "Preparation, characterization, and biological analysis of liposomal formulations of vincristine", Methods Enzymol., 2005; vol. 391, page 40-57;
Urbán P. et al., "A nanovector with complete discrimination for targeted delivery to Plasmodium falciparum-infected versus non-infected red blood cells in Vitro", J Control Release, 2011, vol. 151 (2), pages 202-211); and
Urbán P. et al., "Study of the efficacy of antimalarial drugs delivered inside targeted immunoliposomal nanovectors", Nanoscale Research Letters, 2011, vol. 6, page 620.

## Claims

1. A lipidic nanoparticle comprising 1,2-dioleoyl-3-trimethylammonium-propane (DOTAP), the nanoparticle being conjugated to heparin or a pharmaceutically or veterinary acceptable salt thereof, wherein
the DOTAP is in a percentage by weight comprised from 1 to 30% with respect of the lipidic nanoparticle,
the molar ratio DOTAP:heparin is comprised from 15:1 to 1:20, and
the molar ratio between the total lipid content of the lipidic nanoparticle and heparin is comprised from 50:1 to 5:1.

2. The lipidic nanoparticle according to claim 1, which is a liposome.

3. The lipidic nanoparticle according to any one of the claims 1-2, wherein the percentage by weight of DOTAP is comprised from 4 to 30%.

4. The lipidic nanoparticle according to any one of the claims 1-3, wherein the molar ratio DOTAP:heparin is selected from: 15:1, 2:1, 1.5:1, and 1:15, or, alternatively, it is comprised from 2:1 to 1:1 or it is comprised from 10:1 to 9:1.

5. The lipidic nanoparticle according to any one of the claims 1-4, wherein the heparin is electrostatically conjugated to the surface of the liposome.

6. The lipidic nanoparticle according to any one of claims 1-5, which comprises cholesterol.

7. The lipidic nanoparticle according to any one of claims 1-6, which comprises 1,2-dioleoyl-sn-glycero-3-phosphatidylcholine (DOPC).

8. The lipidic nanoparticle according to any one of the claims 1-7, which encapsulates a drug.

9. The lipidic nanoparticle according to claim 8, wherein the drug is an antimalarial drug.

10. A pharmaceutical or veterinary composition comprising a therapeutically effective amount of the lipidic nanoparticle as defined in any one of the preceding claims, together with other appropriate pharmaceutically or veterinary acceptable excipients and/or carriers.

11. A lipidic nanoparticle according to any one of the claims 1-9 or the pharmaceutical or veterinary composition according to claim 10, for use as a medicine.

12. A lipidic nanoparticle as defined in any one of the claims 1-9 or the pharmaceutical or veterinary composition according to claim 10, for use in the treatment of malaria.

13. Use of the lipidic nanoparticle as defined in any one of the claims 1-9 as drug delivery system.

14. Use of the lipidic nanoparticle as defined in any one of the claims 1-9 for controlling the malaria transmission through mosquito.
